# EUROPEAN PATENT APPLICATION

(11) **EP 1 958 629 A1**
(43) Date of publication of application: **20.08.2008**
(21) Application number: 08002985.3
(22) Date of filing: 12.02.2008
(51) Int. Cl.: A61K 31/355, A61K 9/00, A61P 35/00, A61P 29/00

(54) **A transdermal fluid comprising tocotrienol**

(30) Priority: 13.02.2007 MY 0700208
(71) Applicant: Malaysian Palm Oil Board, 43000 Kajang, Selangor (MY)
(72) Inventor: Nesadurai, Kalanithi, 43000 Kajang Selangor (MY); Selvaduray, Kanga Rani, 43000 Kajang Selangor (MY); Hafid, Sitti Rahma Abdul, 43000 Kajang Selangor (MY); Razak, Ghazali Abdul, 43000 Kajang Selangor (MY); Huat, Ong Thean, 43000 Kajang Selangor (MY)
(74) Representative: Elsy, David

(57) **Abstract**

The present invention relates to a transdermal fluid. The transdermal fluid containing an active amount of a tocotrienol is described, useful for the treatment or prevention of a cancer or a tumour or an inflammatory disorder, particularly breast cancer. The fluid can be used either in combination with known medication or exclusively.

## Description

### FIELD OF INVENTION

The present invention relates to a transdermal fluid.

### BACKGROUND OF INVENTION

Breast cancer worldwide affects nearly one million women per year and although current treatments do help many patients, more than 350,000 die from the disease. In the United States 215,990 women were diagnosed with breast cancer in 2004 and 40,110 died from the disease. In Malaysia, breast cancer is the most frequent cancer amongst women. In 2002, the National Cancer Registry recorded 4,337 new cases of breast cancer in Malaysia, which comprised 30.4% of all cancers in women.

International variation in breast cancer incidence rates and changes in incidence amongst migrant populations have indicated that breast cancer risk is influenced by environmental factors, in particular diet, and therefore preventable.

The established risk factors for breast cancer include a family history of breast cancer, early menarche, late age at first childbirth, late age at menopause and history of benign breast disease. With the exception of the genetic predisposition to the disease the rest of the risk factors point to the life time exposure of women to estrogen. Estrogen does not cause the disease but is involved in the progression and development of breast cancer. Anti-estrogens are therefore used as therapy in the control of breast cancer progression.

Observational studies that have assessed exposure to vitamin E by plasma or adipose tissue concentrations of α-T have failed to provide consistent support for the idea that α-T provides any protection against breast cancer. In contrast, studies in human breast cancer cells indicate that of α-, γ-, δ-T3 have potent anti-proliferative and pro-apoptotic effects that would be expected to reduce the risk of breast cancer whilst α-T had no effect. Galli and co-workers recently demonstrated that γ-T when compared with α-T showed a much stronger inhibitory effect on prostate cancer cell growth. However, when tested further with the T3 homologues he found that γ-T3 had greater anti-proliferative effects than γ-T. Thus it seems plausible that the modest protection from breast cancer associated with dietary vitamin E may be due to the effects of the other T and T3 in the diet.

Scientific investigations have been undertaken to associate possible functional properties, antioxidant or otherwise in the diet, which could be efficient in preventing diseases like cancer. One such antioxidant is vitamin E. Previously eight dietary components α-, β-, γ-, δ-tocopherols (T) and α-, β-, γ-, and δ-tocotrienols (T3) were all considered forms of vitamin E. α-T is thought to be the most biologically important form of vitamin E. Recent guidelines have equated α-T with vitamin E, discounting other tocopherols and the Tocotrienols. Tocopherols and tocotrienols are present in the oil fraction of cereal grains, seeds and nuts. In most food sources, tocopherols are more prevalent than tocotrienols.

### SUMMARY OF INVENTION

Accordingly, there is provided a transdermal fluid containing an active amount of a tocotrienol or derivative thereof or a mixture thereof for a prevention or treatment of a cancer and/or a tumour in a mammal.

Further, there is also provided a transdermal fluid containing an effective amount of a tocotrienol or derivative thereof or a mixture thereof for a prevention or treatment of an inflammatory disorder in mammal.

The present invention consists of several novel features and a combination of parts hereinafter fully described and illustrated in the accompanying drawings, it being understood that various changes in the details may be made without departing from the scope of the invention or sacrificing any of the advantages of the present invention.

### BRIEF DESCRIPTION OF THE ACCOMPANYING DRAWINGS

For the purpose of facilitating and understanding of the present invention, there is illustrated in the accompanying drawings, from an inspection of which, when considered in connection with the following description, the invention, its construction and operation and many of its advantages would be readily understood and appreciated.
FIG. 1 shows δ-T3 measured in the lipid extract obtained from the different tissues;
FIG. 2 shows the 3, (4,5-dimethylthiazole-2-yl)2,5diphenyltetrazolium bromide (MTT) results;
FIG. 3 shows the concentrations of IFN-γ in splenocytes of mice that received δ-T3;
FIG. 4 shows the increase in natural killer cells and B-lymphocytes in mice receiving δ-tocotrienols;
FIG. 5 shows the TNF-Alpha Levels in BALB/c Mice at V3.

### DETAILED DESCRIPTION OF THE PREFERRED EMBODIMENTS

The present invention relates to a transdermal fluid. Hereinafter, this specification will describe the present invention according to the preferred embodiments of the present invention. However, it is to be understood that limiting the description to the preferred embodiments of the invention is merely to facilitate discussion of the present invention and it is envisioned that those skilled in the art may devise various modifications and equivalents without departing from the scope of the appended claims.

In the present invention, an innovative route of delivery of δ-tocotrienol to the breast adipose tissue was examined. Additionally, because the ability of tocotrienols to reduce risk of breast cancer is likely to be determined by their delivery to the breast, concentrations of these dietary constituents found in the breast adipose tissue was determined, and related to transdermal intake.

Among all biological markers of qualitative composition of dietary intake of fatty acids, adipose tissue fatty acid composition is particularly advantageous because it reflects qualitative dietary intake of fatty acids on a long term basis thereby avoiding the potential bias derived from the disease on the measured biochemical parameters. Additionally, because the ability of tocopherol and tocotrienols to reduce risk of breast cancer is likely to be determined by their delivery to the breast, it will be important to determine concentrations of these dietary constituents in the breast adipose tissue, and to relate these concentrations to dietary intake.

The following are the advantages of tocotrienols as compared to commercially available antioestrogen drugs such as Tamoxifen or Anastrozole for breast cancer treatment. Tocotrienols are non-hormonal natural products with no known adverse side effects and over dosage. Tamoxifen has many side effects. Unlike Tamoxifen or Anastrozole, tocotrienols are effective irrespective of the estrogen-receptor status of the breast cancer cell lines. Unlike Tamoxifen which can be consumed for a maximum period of 5 years, tocotrienols have no known time limit. Tocotrienol-induced apoptosis is independent of death receptor apoptotic signalling. However, drugs such as Tamoxifen or Anastrozole become ineffective due to a diminishing response from the death receptors over time.

δ-tocotrienol is the most effective inhibitor of breast cancer cell growth and also has the highest synergistic efficacy with Tamoxifen (the goal standard for breast cancer therapy). Oral administration of tocotrienols ends up with poor uptake into the blood. There is provided herein an alternative method of delivering tocotrienols to the breast.

### Murine Studies

BALB/c mice were divided into 2 groups. Control group only received ethanol the carrier whilst the experimental group was treated with 1mg of δ-tocotrienols daily for 4 weeks. It was applied on the back to examine subcutaneous absorption. The abdominal and breast adipose tissue had the highest absorption after the 1^{st} week (1000 fold) followed by the subcutaneous tissue. Significant changes in tocotrienol levels in blood, spleen and liver were reached by week 4.

The mice were further immunized subcutaneously with ovalbumin (50 µg) adjuvanted in alum. Interferon-gamma (IFN-γ) was measured upon antigen specific stimulation of OVA. There was a significant increase in IFN-γ in mice that received δ-tocotrienols. Single colour flow cytometry analysis using various leukocyte marker antibodies demonstrated that natural killer cells (NK) and B-lymphocytes were significantly increased in the δ-tocotrienol group compared to the control.

This alternative delivery has shown promising results of delivering potent compounds to specific sites at the same time elucidating an effective immune system through a Th1 response.

### Dosage

As used in accordance with the methods of the present invention, tocotrienol compositions are administered to human subjects transdermally, as needed, on a daily basis in single or multiple doses from about 1 mg to 1000 mg at a frequency of about 1-3 doses per day. More preferably, dosages are provided to adult subjects in the range from about 100 mg to 800 mg at a frequency of about 2-3 doses per day. Most preferably, dosages of about 600 mg are provided to adult subjects at a frequency of about 2-3 doses per day.

### Animals

Female BALB/c mice 3 to 4 weeks old were obtained from the Animal Breeding Unit, Institute for Medical Research (IMR), Kuala Lumpur, Malaysia. The care and treatment of the experimental animals conformed to the guidelines of the IMR for the ethical treatment of laboratory animals. The control animals were treated with ethanol alone whilst the experimental groups were applied daily with 0.5ml of a 20mg/ml concentration to deliver 1 mg of δ-tocotrienol. Mice were also immunized subcutaneously with ovalbumin (50 mg) adjuvanted in alum (ratio 1:1) at day 11.

### Analysis of tocotrienols and tocopherols in breast adipose tissue.

The tocopherols and tocotrienols were analysed by HPLC. The system used was a Shimadzu LC-10AT HPLC coupled with a Shimadzu Model RF-10AXL fluorescence spectrophotometer, Shimadzu Class VP data acquisition software, and silica column (YMC A-012/ 150x6 mm I.D, 5µm). The eluting solvent was hexane/isopropyl alcohol (99.5/0.5 v/v) at a flow rate of 2.0 ml/min. The detector was set at excitation wavelength of 295 nm and emission wavelength of 325 nm. 500 mg adipose tissue was homogenized with 4:1:1 mixture of hexane:ethanol and 0.9% sodium chloride at 10000 rpm for 5 minutes. The homogenate was then centrifuged at 2000 rpm for 5 minutes. The resulting supernatant was filtered and evaporated using a rotary evaporator. A known amount of lipid sample was dissolved in a 10 ml volumetric flask using the eluting solvent and 10 µl of the solution was injected into the HPLC system. A standard solution of a mixture of α, γ, and δ-tocotrienols was also injected accordingly. Quantification of the major components of vitamin E was carried out by comparing the peak areas of the components with those of the standards.

### T-Lymphocyte Culture

The experimental mice were sacrificed using ether. Spleen was removed aseptically into RPMI complete medium and single cell suspension was made by teasing out the splenocytes from the spleen capsule. Before culture, cells were stained with 0.4% Trypan Blue Stain (GIBCO, Invitrogen Corp, New Zealand) and cell viability count was performed using a haemocytometer. The spleen cell suspension was diluted to give a 5 x 10⁶ cells/ml solution and Concanavalin A (Sigma-Aldrich Inc, St Louis Missouri, USA) was added at a final concentration of 25µg/ml. 200µl of this suspension was plated on to 96-well flat-bottomed tissue culture plates (Becton Dickinson, USA) and left in a humidified CO₂ incubator at 37°C for 72 hours. At the end of each incubation period, the plate was removed from the incubator and stored at -70°C. The plates were thawed prior to performing ELISA.

### Enzyme-Linked Immunosorbent Assay (ELISA)

The tissue culture plates that were frozen at -30°C were thawed. The samples from the various wells were transferred into appropriately labelled clean Eppendorf tubes. The tubes were centrifuged at 1000 rpm for 10 minutes at 4°C. The supernatant was transferred to fresh appropriately labelled Eppendorf tubes. The pellet was discarded. The supernatant was analysed by ELISA to quantify the amount of IFN-γ and IL-4 produced following the *in vitro* stimulation with Concanavalin A. ELISA was performed using ELISA kits for mouse IFN-γ and IL-4 (BD, Pharmingen) as recommended by the manufacturers.

### Antibody Staining

Fluorescein isothiocyanate (FITC) or Phycoerythrin-labeled antibodies were diluted by adding 20µl of antibody to 50µl of Staining Buffer in separate tubes. 100µl of whole blood from mouse was added to each tube and mixed gently. The samples were then incubated for one hour at room temperature in the dark. After incubation, 2ml of RBC Lysis Buffer was added into each tube and mixed gently. Samples were then centrifuged at 400g for 5 minutes at room temperature. A wash step was carried out by adding 2ml of Staining Buffer and samples were again centrifuged at 400g for 5 minutes at room temperature. Supernatant was removed and 200µl Staining Buffer was added into each tube followed by 200µl of 4% Formalin. Samples were analysed using Flow Cytometer.

### 3, (4,5-dimethylthiazole-2-yl)2,5diphenyltetrazolium bromide (MTT)

Viability of the cells was measured by the MTT assay. In this assay MTT is converted to a blue formazan product by dehydrogenases that are active in living cells. Splenocytes were seeded with concanavalin A or OVA in a 96 well plate in a total volume of 200µl of medium. MTT (25µl of 5mg/ml) was added to each well. After 4 hours, 100µl of extraction buffer consisting of 20% SDS dissolved in a 50% dimethyl formamide/50% water solution at pH 4.0 was added. The blue colour formed was measured at 590 nm.

### δ-tocotrienol composition in adipose tissue

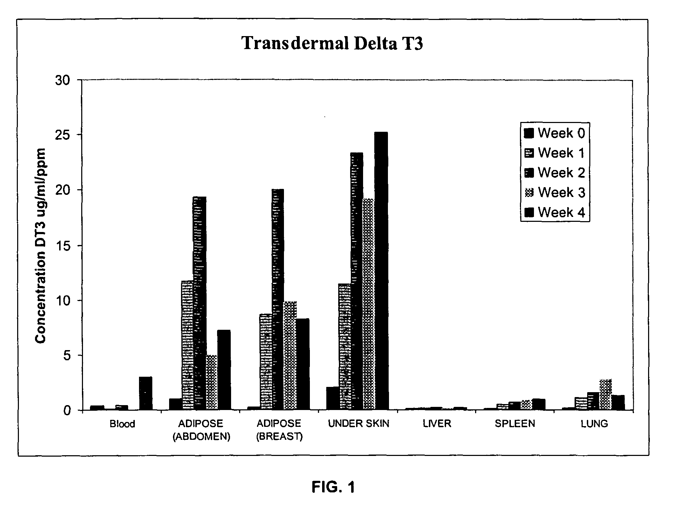

FIG. 1 shows δ-T3 measured in the lipid extract obtained from the different tissues. Breast and abdominal adipose and subcutaneous fat tissue show significant increases following transdermal application starting from week 1 and reaching optimum concentration at week 2. There was a drop in concentration at week 3. Changes in blood, liver and spleen levels were only seen at week 4.

### MTT

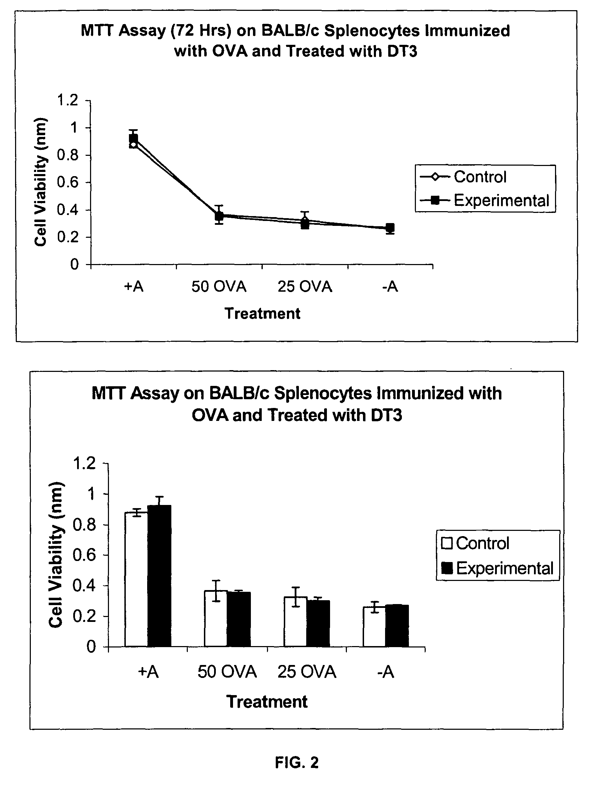

FIG. 2 shows the MTT results. The MTT assay uses the principle whereby cell growth or cell death is indicated by the conversion or lack of conversion of the tetrazolium salt to the coloured product, formazan, the concentration of which can be measured spectrophotometrically. As can be seen from FIG. 2, both control and experimental splenocytes grew normally (no change in OD reading) with the addition of concanavalin A or OVA.

### δ-tocotrienol stimulates Interferon-γ production upon vaccination

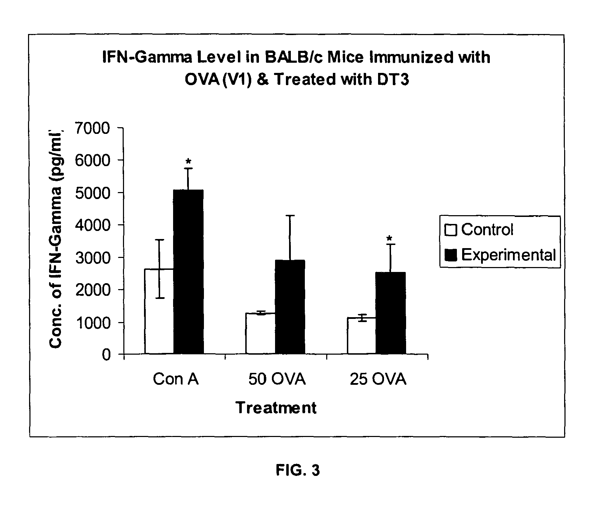

To assess the effects of δ-T3 on immune cell activity in mice immunized with OVA, all mice were examined and compared levels of IFN-γ and IL-4 after different treatments. As shown in FIG. 3, concentrations of IFN-γ in splenocytes of mice that received δ-T3 and were immunized with OVA were significantly higher than those of control mice.

### Antibody responses

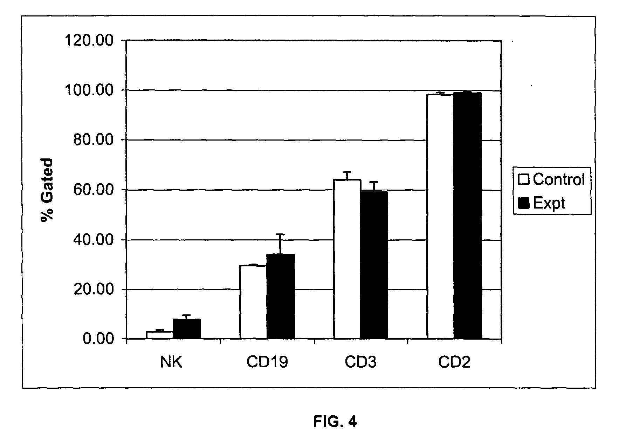

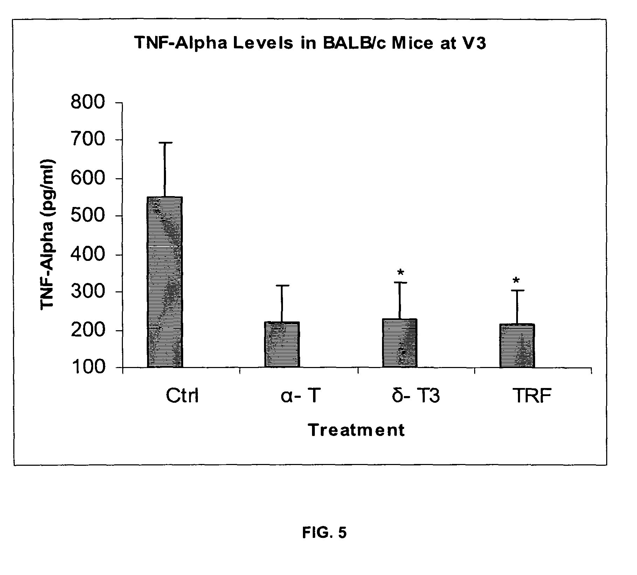

FIG. 4 shows the increase in natural killer cells and B-lymphocytes were seen in mice receiving δ-tocotrienols whereas FIG. 5 shows the TNF-Alpha Levels in BALB/c Mice at V3.

Very little is known about the absorption of tocotrienols. The body's preference for a-tocopherol is established. Furthermore it has been shown that dietary α-T decreases α-T3 but not γ-T3 in rats. It is interesting to note that tocotrienols are preferentially distributed to adipose tissue and the skin. The concentrations of α-T3 in these tissues were observed to be significantly higher than α-T when the rats were fed 50mg of α-T3 or α-T respectively. This perhaps provide evidence that the tocotrienols are distributed via the lymphatic system to these tissues or there maybe unknown α-TTP independent pathways for distribution, in view that the observation cannot be explained by the α-TTP affinity mechanism.

### Human Studies

The study population was derived from Kuala Lumpur Hospital and was approved by the Ethics Committee of the Ministry of Health, Malaysia. Of the 75 women studied, 40 had breast cancer and 35 had benign breast pathologies. Both groups of women presented with breast lumps. Both groups had imaging and needle biopsies done to prove the pathology i.e. benign vs. malignant prior to surgery and the adipose tissue from the breast was obtained at surgery. The tissue submitted was not the actual pathology but from the surrounding adipose tissue. After the initial pathological evaluation of the specimen, 0.2-1.0 g of adipose tissue was washed with saline, frozen immediately in liquid nitrogen, and stored at -70°C in vials until analysis.

### Adipose tissue preparation and fatty acid analysis

Total lipids were extracted from adipose tissue with chloroform-methanol 2:1 (v:v). The extract was washed with sodium chloride and the mixture was allowed to separate into two phases. The chloroform layer was transferred to a tube and evaporated to dryness under nitrogen. The lipid extract was dissolved in 200 µl of chloroform-methanol 2:1 (v:v) and directly used for column procedure.

Triacylglycerol was purified by adsorption chromatography on silica tubes (Supelco, France) as follows: the column was pretreated with 2 ml chloroform-methanol 2:1 (v:v). Then the lipid sample was applied to the column and the tube treated with 100 µl of this same system solvent added to the column. Triacyglycerols were eluted with 20 ml of chloroform. Chloroform fractions were collected in screw-cap tubes with a Teflon seal. Fractions obtained were evaporated to dryness. Three hundred microlitres of sodium methoxide 2 N and 500 µl of boron trifluoride were added to convert the fatty acids to their methyl esters. The mixture was incubated and shaken 15 min at room temperature. Fatty acid methyl esters were extracted twice into hexane.

Fatty acid methyl esters (FAME) composition was determined by capillary gas chromatography. The system was composed of a GC 800 series chromatograph (Perkin Elmer, USA) equipped with a cold on-column injector, and a flame ionization detector. A 60 m long x 0.22 mm internal diameter fused silica column (Varian, USA) was used. Helium was used as the carrier gas at a flow rate of 1.5 ml/min. The detector temperature was 280°C. One microlitre was injected through the cold on-column injector (60°C). The oven temperature was programmed to rise from 60-170°C at a rate of 15°C/min and kept constant for 20 min, from 170-190°C at a rate of 5°C and kept constant for 10 min, and from 190-215°C at a rate of 1°C/min and kept constant for 10 min. Identification of FAME was obtained by comparison of their relative retention times with those pure standard mixtures. The relative amount of each fatty acid was quantified by integrating the peak at baseline and dividing the results by the total area for all fatty acids. All integrations were performed by the same laboratory worker. Peaks accounting for less than 1% of total area were well detected and quantified.

### Analysis of tocotrienols and tocopherols in breast adipose tissue

The tocopherols and tocotrienols were analysed by HPLC. The system used was a Shimadzu LC-10AT HPLC coupled with a Shimadzu Model RF-10AXL fluorescence spectrophotometer, Shimadzu Class VP data acquisition software, and silica column (YMC A-012/ 150x6 mm I.D, 5µm). The eluting solvent was hexane/isopropyl alcohol (99.5/0.5 v/v) at a flow rate of 2.0 ml/min. The detector was set at excitation wavelength of 295 nm and emission wavelength of 325 nm. 500 mg adipose tissue was homogenized with 4:1:1 mixture of hexane:ethanol and 0.9% sodium chloride at 10000 rpm for 5 minutes. The homogenate was then centrifuged at 2000 rpm for 5 min. The resulting supernatant was filtered and evaporated using a rotary evaporator. A known amount of lipid sample was dissolved in a 10 ml volumetric flask using the eluting solvent and 10 µl of the solution was injected into the HPLC system. A standard solution of a mixture of α, γ, and δ-tocotrienols was also injected accordingly. Quantification of the major components of vitamin E was carried out by comparing the peak areas of the components with those of the standards.

### Statistics

Mean and standard deviations of individual fatty acids were calculated, and the total sum of fatty acids in each family was determined. Statistical analysis was performed using the SPSS program. Two-sided Student's t-tests were used to compare the mean differences between women with and without breast cancer in relation to fatty acid composition (FAC) and vitamin E levels in adipose tissue. Results were considered significant at *p*<0.05.

### Results

### Fatty acid composition in breast adipose tissue

Table 1 below shows the fatty acid composition of breast fat in benign and malignant lumps. The major fatty acids in breast adipose tissue were oleic acid (18:1 n-9c), palmitic acid (16:0), linoleic acid (18:2 n-6) and stearic acid (18:0). These fatty acids accounted for about 90% of total area under the chromatographic curve. There was no significant difference in the FAC of breast adipose tissue from benign and malignant lumps.

**Table 1.**

| **Fatty acids** | | **Benign Mean value % (± SD) *n*=35** | **Malignant Mean value % (± SD) *n*=40** |
|---|---|---|---|
| **Saturates** | | | |
| | 12:0 | 1.23 (0.35) | 0.91 (0.44) |
| | 14:0 | 2.25 (0.46) | 2.21 (0.55) |
| | 16:0 | 29.93 (0.95) | 28.43 (1.97) |
| | 18:0 | 2.98 (1.29) | 4.83 (0.97) |
| | 20:0 | 0.19 (0.09) | 0.51 (0.30) |
| | **Total** | **36.58 (0.63)** | **36.89 (0.84)** |
| **Monounsaturates** | | | |
| | 16:1 n-7c | 5.52 (2.11) | 3.43 (1.19) |
| | 18:1 n-9c | 46.30 (0.93) | 45.65 (2.34) |
| | 20:1 n-9 | 0.51 (0.04) | 0.49 (0.18) |
| | **Total** | **52.33 (1.02)** | **49.57 (1.24)** |
| **n-6 PUFA** | | | |
| | 18:2 n-6 | 10.82 (0.89) | 12.45 (1.05) |
| | 18:3 n-6 | 0.26 (0.06) | 0.28 (0.11) |
| | **Total** | **11.08 (0.48)** | **12.73 (0.58)** |

### Vitamin E composition in breast adipose tissue

Table 2 below shows the T and T3 measured in the lipid extract of breast adipose tissue obtained from 40 patients with malignant and 35 with benign breast lumps. The content of T and T3 was expressed in µg/g adipose tissue. In malignant lumps, the mean content of α-T in breast adipose tissue was 126.2 µg/g adipose tissue whilst in the benign lumps it was 147.0 µg/g. There was a large variability between patients especially for α-T. This could be due to different dietary intake patterns as tocopherols are prevalent in many food items. The mean of α-T value in adipose tissue was however not significantly different in malignant cancer patients than in benign subjects (*p*=0.435). The mean γ-T (7.51 vs. 6.18) and δ-T (1.26 vs. 0.65) levels were also reduced in malignant patients as compared to benign. Whilst it was not significant for γ-T (*p*=0.363), the reduced level of δ-T was significant (*p*=0.033).

**Table 2**

| **Parameters measured** | **Benign Mean value (µg/g) (±SD) n=35** | **Malignant Mean value (µg/g) (±SD) n=40** |
|---|---|---|
| **Tocopherols** | | |
| α | 147.04 (61.90) | 126.17 (80.94) |
| β | 2.75 (1.57) | 2.81 (1.53) |
| γ | 7.51 (3.79) | 6.18 (4.17) |
| δ | 1.26 (0.85) | 0.65 (0.31) |
| **Total** | **158.56 (65.41)** | **135.81 (85.12)** |
| **Tocotrienols** | | |
| α | 11.35 (3.31) | 7.21 (4.28) |
| γ | 7.90 (2.61) | 6.03 (2.47) |
| δ | 0.82 (0.29) | 0.49 (0.31) |
| **Total** | **20.07 (6.02)** | **13.73 (6.09)** |

In breast cancer patients, the mean content of α-, γ-, and δ-T3 in breast adipose tissue was 13.73 µg/g adipose tissue. In patients with benign lumps it was 20.0µg/g adipose tissue. Mean T3 value was significantly (*p*= 0.006) lower in breast cancer patients than in subjects with benign lumps. There was a decrease in α-T3 (*p*=0.006), γ-T3 (*p*=0.047), and δ-T3 (*p*=0.018) in malignant tissue compared to benign. The distribution of tocotrienols with α and γ-T3 being higher also reflects closely the composition of tocotrienols in palm oil.

The evaluation of long-term nutritional vitamin E status in breast cancer patients using adipose tissue concentrations has definite advantages over measurement of plasma levels. Plasma levels of tocopherol and tocotrienols change very rapidly in humans following modifications in the dietary intake, reaching new steady state levels within a few days. The content of α-T in adipose tissue has been evaluated in humans in relation to dietary intake of vitamin E and it has been shown that α-T content reflects long-term dietary intake of vitamin E.

Several studies that carefully collected adipose tissue have investigated the relationship between adipose tissue tocopherols and breast cancer risk. Overall, studies of the association of vitamin E with breast cancer risk suggest the possibility that increased dietary exposure to vitamin E may slightly reduce breast cancer risk. However, there is no evidence that supplemental vitamin E, most, if not all of which is in the form of α-T, confers any protection at all. Furthermore, cell culture data has showed that α-T combined with tamoxifen increased the IC₅₀ for tamoxifen in MCF-7 cells more than 1000 fold and in a further study α-T completely blocked the potent growth inhibitory effects of tamoxifen on MDA-MB-231 cells. In contrast, it has been shown that α-, γ-, and δ-T3 and the tocotrienol rich-fraction of palm oil inhibited proliferation of MCF-7(12) and in ZR-75-1 cells, both in the absence and the presence of estradiol and tamoxifen. The inhibitory effect on cell growth was more pronounced with γ and δ-T3. The mechanism of action is unknown, with previous data suggesting action does not reside in antagonism of estrogen action or in alterations to growth inhibitory insulin-like growth factor binding proteins in MCF-7 human breast cancer cells. Tocotrienols are also reported to have a pro-apoptotic effect on several tumour cell lines. However, McIntyre and co-workers have also shown that highly malignant cells are more sensitive to the anti-proliferative and apoptotic effects of tocotrienols in comparison with pre-neoplastic cells.

The fatty acid composition data reflects closely the intake of a palm oil diet in Malaysia. This is not surprising as it is the cheapest and most readily available oil in the country and is reported to constitute the major fat in the diet of the majority of Malaysians. (31) While most vegetable oils provide mainly α- or γ-T, palm oil is unique in the sense that it contains relatively large concentrations of T3. The distribution of the various T and T3 fractions in palm oil are as follows: α-T 32%, α-T3 25%, γ-T3 29% and δ-T3 14%. In the United States, analyses of balanced diets ranging from 2,000 to 3,000 kcal per day indicated that the average daily intakes of vitamin E range from 7 to 11 mg. Malaysian data on vitamin E intakes are presently limited. However, it is envisaged that for a 2,000 to 3,000 kcal per day diet one would be consuming between 10 to 15 mg of tocotrienols per day.

The most significant finding of the present study was the higher, 65%-more tocotrienol (α-T3, γ-T3 and δ-T3) concentrations in the adipose tissue of the benign lumps in comparison to the women with malignant breast lumps. There was no difference in the α-T and γ-T content. However, δ-T showed a significant reduction in malignant vs. benign adipose tissue. The depletion in tocotrienols in patients with malignant breast lumps compared to patients with benign lumps could be due to its role as an antioxidant in quenching free radicals and regulating peroxidation reactions.

It is noted that the amount of the tocotrienol composition administered for the treatment or prevention of cancer or inflammatory disorder in a mammal on a daily basis is 5µg to 1000mg.

Tocotrienols possess powerful antioxidant-, anticancer- and cholesterol-lowering properties. Some studies have confirmed that tocotrienol activity as an antioxidant-, anticancer- and cholesterol-reducing substance to be stronger than tocopherols. Tocotrienols are thought to have more potent antioxidant properties than α-T. The unsaturated side-chain of tocotrienol allows far more efficient penetration into tissues, such as the adipose, brain and liver, that have saturated fatty layers. Experimental research examining the antioxidant, free radical scavenging effects of tocopherols and tocotrienols revealed that tocotrienols appear superior because of their better distribution in the fatty layers of the cell membrane.

Oxidative stress has also been implicated in breast cancer and may influence breast cancer by altering gene expression or by promoting oxidative DNA damage. Although the hydroperoxide levels in the breast adipose tissue were not measured, prior art documents have demonstrated higher conjugated dienes and hydroperoxides in breast adipose tissue of breast cancer patients than in control patients.

Palm oil contains high amounts of tocotrienols and it has been previously demonstrated *in vitro* and *in vivo* that tocotrienols protect against breast cancer. Thus it seems plausible that the modest protection from breast cancer associated with dietary vitamin E maybe due to the effects of tocotrienols in the diet.

The low tocotrienols in the adipose tissue of malignant breast lumps needs to be further investigated as to whether their intake of tocotrienols was low or whether it was used up because they had cancer. Studies have shown that tocotrienol supplementation up to 240 mg for 16-months duration does not have any apparent adverse effect. (43) Furthermore, it is likely that breast adipose tissue concentrations will be five- to 10-fold those in plasma. This indicates that lower levels of tocotrienol supplementation might be adequate to reach breast adipose tissue tocotrienol concentrations similar to those that inhibit proliferation and promote apoptosis in breast cancer cells.

## Claims

1. A transdermal fluid containing an active amount of a tocotrienol or derivative thereof or a mixture thereof for a prevention or treatment of a cancer and/or a tumour in a mammal.

2. The transdermal fluid of claim 1, wherein said cancer is breast cancer.

3. The transdermal fluid of claim 1 or 2 which comprises α-tocotrienol, and/or β-tocotrienol, and/or γ-tocotrienol and/or δ-tocotrienol, preferably δ-tocotrienol, or derivatives of these, or combinations of one or more of these.

4. The transdermal fluid of claim 1, 2 or 3 wherein the amount of the tocotrienol composition administered on a daily basis is 5µg to 1000mg.

5. The transdermal fluid of claim 1, 2 or 3 wherein the tocotrienol composition is used either in combination with known medication or used exclusively.

6. A use of an effective amount of a transdermal fluid of claims 1-5 in the manufacture of a medicament for a prevention or treatment of a cancer or a tumour in a mammal, preferably breast cancer.

7. A use of claim 6 wherein the fluid is applied topically.

8. A method of preventing or treating a cancer and/or a tumour in a mammal comprising administering transdermally to said mammal an effective amount of a fluid of claims 1-5.

9. A transdermal fluid containing an effective amount of a tocotrienol or derivative thereof or a mixture thereof for a prevention or treatment of an inflammatory disorder in mammal.

10. The transdermal fluid of claim 9 which comprises α-tocotrienol, and/or β-tocotrienol, and/or γ-tocotrienol and/or δ-tocotrienol, preferably δ-tocotrienol, or derivatives of these, or combinations of one or more of these.

11. The transdermal fluid of claim 9 or 10 wherein the amount of the tocotrienol composition administered on a daily basis is 5µg to 1000mg.

12. The transdermal fluid of claim 9, 10 or 11 wherein the tocotrienol composition is used either in combination with known medication or used exclusively.

13. A use of an effective amount of a fluid of claims 9-12 in the manufacture of a medicament for a prevention or treatment of an inflammatory disorder in a mammal.

14. A use of claim 13 wherein the fluid is applied topically.

15. A method of treating or preventing an inflammatory disorder in a mammal comprising administering transdermally to said mammal an effective amount of a fluid of claims 9-12.
